# EUROPEAN PATENT SPECIFICATION

(11) **EP 1 301 475 B1**
(45) Date of publication and mention of the grant of the patent: **22.11.2006**
(21) Application number: 01950200.4
(22) Date of filing: 19.07.2001
(51) Int. Cl.: C07C 311/51, A61K 31/18, A61P 3/00

(54) **ARYL SULFONAMIDES AS SEROTONIN ANTAGONIST FOR THE TREATMENT OF OBESITY**
ARYL-SULFONAMIDE ALS SEROTONIN-ANTAGONISTEN FÜR DE BEHANDLUNG VON FETTLEIBIGKEIT
ARYLES SULFONAMIDES EN TANT QU'ANTAGONISTE DE LA SEROTONINE POUR TRAITER L'OBESITE

(30) Priority: 21.07.2000 SE 0002739
(43) Date of publication of application: 16.04.2003
(73) Proprietor: BIOVITRUM AB, 112 76 Stockholm (SE)
(72) Inventor: CALDIROLA, Patrizia, S-756 46 Uppsala (SE); JOSSAN, Sukhwinder, S-756 47 Uppsala (SE); SAKARIASSEN, Kjell, S., I-10015 IVREA (IT); SVARTENGREN, Jan, S-753 26 Uppsala (SE)
(86) International application number: PCT/SE2001/001652
(87) International publication number: WO 2002/008179

(56) References cited:
- EP-A1- 0 611 003
- EP-A1- 0 815 861
- EP-A1- 1 072 597
- WO-A1-98/27081
- WO-A1-98/29411
- WO-A2-99/02502
- WO-A2-99/42465
- PATENT ABSTRACTS OF JAPAN & JP 11 171 856 A (FUJIREBIO INC) 29 June 1999
- THERESA A. BRANCHEK ET AL.: '5-HT6 receptor as emerging targets for drug discovery' ANNU. REV. PHARMACOL. TOXICOL. vol. 40, 2000, pages 319 - 334, XP002967462
- Br. J. Pharmac., 1999, Suppl. 126, P66, Bentley et al.

## Description

### TECHNICAL FIELD

The present invention relates to the use of aryl sulfonamide compounds, active as 5-HT₆ receptor antagonists, in the treatment of obesity.

### BACKGROUND ART

Obesity is a condition characterized in an increase in body fat content resulting in excess body weight above accepted norms. Obesity is the most important nutritional disorder in the western world and represents a major health problem in all industrialized countries. This disorder leads to increased mortality due to increased incidences of diseases such as cardiovascular disease, digestive disease, respiratory disease, cancer and NIDDM (type II diabetes). Searching for compounds, which reduce body weight has been going on for many decades. One line of research has been activation of serotonergic systems, either by direct activation of serotonin receptor subtypes or by inhibiting serotonin reuptake. The exact receptor subtype profile required is however not known.

Serotonin (5-hydroxytryptamine or 5-HT), a key transmitter of the peripheral and central nervous system, modulate a wide range of physiological and pathological functions, including anxiety, sleep regulation, aggression, feeding and depression. Multiple serotonin receptor subtypes have been identified and cloned. One of these, the 5-HT₆ receptor, was cloned by several groups in 1993 (M Ruat, E Traiffort, J-M Arrang, J Tardivel-Lacombe, J Diaz, R Leurs, J-C Shwartz. *Biochem. Biophys. Res. Commun.* 1993, 193 (1) 268-276; M Sebben, H Ansanay, J Bockaert, A Dumuis, *NeuroReport* 5, 2553-2557 (1994).) This receptor is positively coupled to adenylyl cyclase and displays affinity for antidepressants such as clozapine. Recently, the effect of 5-HT₆ antagonist and 5-HT₆ antisense oligonucleotides to reduce food intake in rats has been reported (JC Bentley, CA Mardsen, AJ Sleight and KC Fone Effect of 5-HT₆ antagonist Ro 04-6790 on food consumption in rats traineds to a fixed feeding regime *Br J Pharmac.* 1999 Suppl 126 P66; JC Bentley, AJ Sleight, CA Mardsen, KCF Fone 5-HT₆ antisense oligonucleotide ICV affects rat performance in the water maze and feeding *J Psychopharmacol Suppl* A64 1997 255).

Aryl sulfonamide compounds have been disclosed as possessing 5-HT₆ receptor activity and being useful in the treatment of CNS disorders (EP 815861). Further classes of aryl sulfonamide compounds with 5-HT₆ receptor activity have been reported in WO 98/27081, WO 99/02502 and WO 99/42465. The compounds are believed to be of potential use in the treatment of certain CNS disorders.

The document WO 98/29411 discloses sulfonamide compounds having 5-HT receptor activity. These compounds are indicated to have utility in the treatment of CNS disorders, for example depression, anxiety, psychoses (for example schizophrenia), tardive dyskinesis, Parkinson's disease, obesity, hypertension, Tourette's syndrome, sexual dysfunction, drug addiction, drug abuse, cognitive disorders, Alzheimer's disease, senile dementie, obsessive-compulsive behavious, panic attacks, social phobias, eating disorders and anorexia, cardiovascular disease, cerebrovascular disorders, non-insulin dependent diabetes meelitus, hyperglycaemia, constipation, arrhythmia, disorders of the endocrine system, stress and spasticity.

The document EP 0 611 003 discloses substituted phenyl sulfonamides as selective B3 agonists for the treatment of diabetes and obesity.

The object of the present invention is to present an improved method of treatment of obesity. A further object is a new use of compounds for the manufacture of medicaments for obesity treatment.

### BRIEF DESCRIPTION OF THE DRAWING

Fig. 1
   Effect of SB-271046 (5-Chloro-3-methyl-benzo-[b]thiophene-2-sulphonic acid (4-methoxy-3-piperazin-yl-phenyl)-amide monohydrochloride) on food intake in ob/ob mice. mCPP (m-chloro-phenylpiperazine) was used as a positive control.

### SUMMARY OF THE INVENTION

The objects of the invention are achieved by the use of a compound of formula (I) or formula (II) 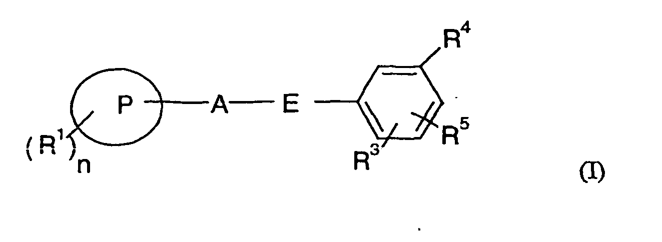 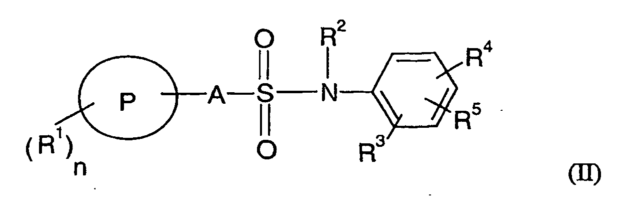 wherein
E is -SO₂NH- or -NHSO₂-;
R² is hydrogen, C₁₋₆ alkyl or arylC₁₋₆alkyl;
P is phenyl, naphthyl a bicyclic heterocyclic ring or is a 5- to 7-membered heterocyclic ring each containing 1 to 4 heteroatoms selected from oxygen, nitrogen or sulfur;
A is a single bond, a C₁₋₆ alkylene or a C₁₋₆ alkenylene group;
R¹ is halogen, C₁₋₆ alkyl, optionally substituted by one or more halogen atoms, C₃₋₆ cycloalkyl, COC₁₋₆ alkyl, C₁₋₆ alkoxy, OCF₃, hydroxy, hydroxy C₁₋₆alkyl, hydroxy C₁₋₆alkoxy, C₁₋₆ alkoxy C₁₋₆ alkoxy, C₁₋₆ alkanoyl, acyl, nitro, amino, alkylamino or dialkylamino, cyano or SR¹¹ where R¹¹ is hydrogen or C₁₋₆ alkyl or R¹ is phenyl, benzyl, naphthyl, a bicyclic heterocyclic ring, or is a 5 to 7-membered heterocyclic ring, each containing 1 to 4 heteroatoms selected from oxygen, nitrogen or sulfur;
n is 0, 1,2,3,4,5 or 6;
R³ is a group R⁵ or together with R⁵ forms a group (CH₂)₂O or (CH₂)₃O or R³ is linked to R² to form a group (CH₂)₂ or (CH₂)₃;
R⁴ is -X(CH₂)p-R⁶ wherein
   X is a single bond, CH₂, O, NH or N-C₁₋₆-alkyl;
   p is 0 to 6 and
   R⁶ is an optionally substituted 5- to 7-membered heterocyclic ring containing 1 to 3 heteroatoms selected from nitrogen, sulfur or oxygen, or R⁶ is NR⁷R⁸ wherein R⁷ and R⁸ are independently hydrogen, C₁₋₆ alkyl or aryl C₁₋₆ alkyl, or
R⁴ is selected from a group of formula (i), (ii) or (iii) 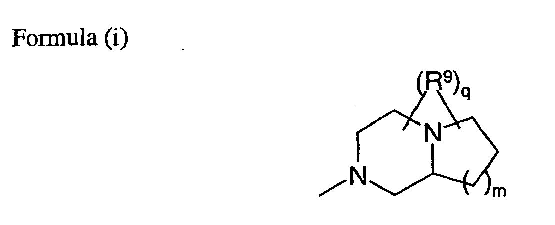
wherein
R⁹ is C₁₋₆alkyl, or C₁₋₆alkyl substituted by one or more halogen atoms;
m is 0, 1 or 2;
q is 0, 1, 2, 3 or 4; or 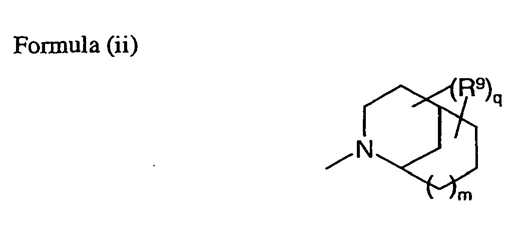
wherein
R⁶, m and q are as defined in formula (i); or 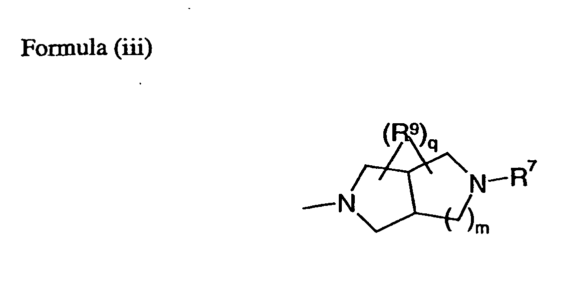
wherein
R⁹, m and q are as defined in formula (i) and R⁷ is hydrogen or C₁₋₆alkyl;
R⁵ is hydrogen, halogen, C₁₋₆alkyl, C₁₋₆alkoxy, optionally substituted with one or more fluorine atoms, C₃₋₆ cycloalkyl, COC₁₋₆ alkyl, C₁₋₆ alkoxy, hydroxy, hydroxy C₁₋₆alkyl, hydroxy C₁₋₆alkoxy, C₁₋₆alkoxyC₁₋₆alkoxy, acyl, nitro, trifluoromethyl or together with R³ forms a group (CH₂)₂O or (CH₂)₃O, cyano or aryl, or pharmaceutically acceptable salts thereof, in the manufacture of a medicament for the treatment of obesity.

Preferably, in formula (I):
R¹ is halogen, C₁₋₆ alkyl, optionally substituted by one or more fluorine atoms, C₃₋₆ cycloalkyl, C₁₋₆ alkoxy, OCF₃, C₁₋₆ alkoxy C₁₋₆ alkoxy, C₁₋₆ alkanoyl, amino, alkylamino or dialkylamino, SR¹¹ where R¹¹ is hydrogen or C₁₋₆ alkyl or R¹ is phenyl, benzyl, naphthyl, a bicyclic heterocyclic ring, or is a 5 to 7-membered heterocyclic ring, each containing 1 to 4 heteroatoms selected from oxygen, nitrogen or sulfur;
n is 0, 1, 2, 3, 4 or 5;
R³ is a group R⁵ or together with R⁵ forms a group (CH₂)₂O or (CH₂)₃O;
R⁴ is selected from a group of formula (i), (ii) or (iii) as mentioned above;
R⁵ is hydrogen, halogen, C₁₋₆alkyl, C₁₋₆alkoxy, optionally substituted with one or more fluorine atoms, trifluoromethyl or together with R³ forms a group (CH₂)₂O or (CH₂)₃O.

Preferably, in formula (II):
R¹ is halogen, C₁₋₆alkyl optionally substituted by one or more halogen atoms, C₃₋₆ cycloalkyl, COC₁₋₆ alkyl, C₁₋₆alkoxy, OCF₃, hydroxy, hydroxy C₁₋₆alkyl, hydroxy C₁₋₆alkoxy, C₁₋₆alkoxyC₁₋₆alkoxy, acyl, nitro, amino, alkylamino or dialkylamino, cyano or R¹ is phenyl, naphthyl, a bicyclic heterocyclic ring or is a 5 to 7-membered heterocyclic ring, each containing 1 to 4 heteroatoms selected from oxygen, nitrogen or sulfur;
R⁴ is -X(CH₂)p-R⁶ where X is a single bond, CH₂, O, NH or N-C₁₋₆-alkyl and p is 0 to 6 and R⁶ is an optionally substituted 5- to 7-membered heterocyclic ring containing 1 to 3 heteroatoms selected from nitrogen, sulfur or oxygen, or R⁶ is NR⁷R⁸ where R⁷ and R⁸ are independently hydrogen, C₁₋₆ alkyl or aryl C₁₋₆ alkyl and
R⁵ is hydrogen, halogen, C₁₋₆ alkyl, C₃₋₆ cycloalkyl, COC₁₋₆ alkyl, C₁₋₆ alkoxy, hydroxy, hydroxy C₁₋₆alkyl, hydroxy C₁₋₆alkoxy, C₁₋₆alkoxyC₁₋₆alkoxy, acyl, nitro, trifluoromethyl, cyano or aryl.

The compounds of formula (I) and (II) can also be used in the form of pharmaceutically acceptable salts thereof.

### DETAILED DESCRIPTION OF THE INVENTION

According to the present invention, it has surprisingly been found that 5-HT₆ receptor antagonists, belonging to the aryl sulfonamide compounds disclosed in WO 98/27081 and WO 99/42465, reduce food intake and body weight. An improved method of treating obesity is therefore provided by the present invention.

In the formulas the alkyl groups may be straight chained or branched both alone and as part of another group. Preferred alkyl groups are methyl and ethyl. "Halogen" means a group selected from fluorine, chlorine, bromine or iodine.

When the group P is a bicyclic heterocyclic ring suitable examples include benzothienyl, indolyl, quinolinyl or isoquinolinyl. When P is a 5 to 7- membered heterocyclic ring suitable examples include thienyl, furyl, pyrrolyl, triazolyl, diazolyl, imidazolyl, oxazolyl, thiazolyl, oxadiazolyl, isothiazolyl, isoxazolyl, thiadiazolyl, pyridyl, pyrimidyl, pyrrolidinyl and pyrazinyl. The heterocyclic rings can be linked to the remaining molecule via any suitable carbon atom or, when present, a nitrogen.

Preferably P is phenyl, naphthyl, thienyl and most preferably benzothienyl. Suitably A is a single bond, a methylene or ethylene group or a -CH=CH- group. Preferably A is a single bond or methylene.

Preferably R¹ is halogen, or C₁₋₆ alkyl optionally substituted by one or more halogen atoms, for example methyl or trifluoromethyl. When R¹ is a heterocyclic group suitable examples include those listed above for P. Preferably n is 0, 1, 2 or 3, particularly 1 or 2.

Suitably R² is hydrogen or C₁₋₆ alkyl. Preferably R² is hydrogen.

It will be appreciated that when R³/R⁵ groups are linked together the two groups must be attached to adjacent carbon atoms of the phenyl ring. Preferably R³ is a group R⁵, in particular hydrogen.

In formula (I) R4 is preferably a group:

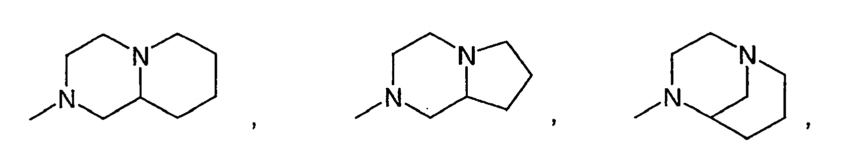

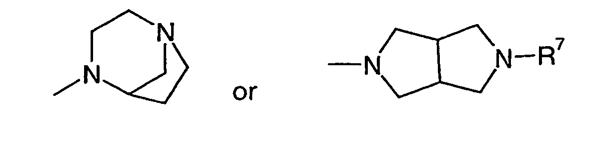

Preferably R⁵ is C₁₋₆ alkoxy, most preferably methoxy. Preferably R⁵ is para with respect to the sulfonamide linkage.

In formula (II) R⁴ is preferably meta with respect to the sulfonamide linkage. Preferably X is a bond, p is 0 and R⁶ is an optionally substituted 5- to 7-membered heterocyclic ring. The heterocyclic rings can be linked to the remaining molecule via a carbon atom or, when present, a nitrogen atom. Optional substituents for these rings, which can be present on carbon and /or nitrogen atoms, include C₁₋₆ alkyl, in particular methyl. More preferably R⁴ is N-piperazine optionally substituted by C₁₋₆ alkyl, particularly unsubstituted piperazine.

A preferred meaning for P-A is benzo[b]thiophen-2yl or benzo[b]thiophen-3-yl optionally substituted by one or two R¹ groups, especially 5-chloro-3-methylbenzo [2] thiophen-2-yl.

A particularly preferred compound of the invention is 5-chloro-*N*-(4-methoxy-3-piperazin-1-yl-phenyl)-3-methyl-2-benzothiophenesulfonamide..

The compounds of the formula (I) and (II) can form acid addition salts with acids such as conventional pharmaceutically acceptable acids, for example maleic, hydrochloric, hydrobromic, phosphoric, acetic, fumaric, salicylic, citric, lactic, mandelic, tartaric and methanesulfonic.

Compounds of formula (I) and (II) may also form solvates such as hydrates and the invention also extends to these forms. When referred to herein, it is understood that the term "compound of formula (I) and (II)" also includes these forms.

Certain compounds of formula (I) and (II) are capable of existing in stereoisomeric forms including diastereomers and enantiomers and the invention extends to each of these stereoisomeric forms and to mixtures thereof including racemates. The different stereoisomeric forms may be separated one from the other by the usual methods. Any given isomer may be obtained by stereospecific or asymmetric synthesis. The invention also extends to any tautomeric forms and mixtures thereof.

The compounds used in the invention are prepared according to the methods described in WO 98/27081 and WO 99/42465.

According to the present invention the compounds for obesity treatment can conveniently be administered in a pharmaceutical composition containing the compound in combination with a suitable excipient. Such pharmaceutical compositions can be prepared by methods and contain excipients which are well known in the art. A generally recognized compendium of such methods and ingredients is Remington's Pharmaceutical Sciences by E.W. Martin (Mark Publ. Co., 15^{th} Ed., 1975). The compounds and compositions can be administered orally, parenterally (for example, by intravenous, intraperitoneal or intramuscular injection), topically, or rectally. Preferably the compounds are administered orally.

For oral therapeutic administration, the active compound may be combined with one or more excipients and used in the form of ingestible tablets, buccal tablets, troches, capsules, elixirs, suspensions, syrups, and wafers. Such compositions and preparations should contain at least 0.1% of active compound. The percentage of the compositions and preparations may, of course, be varied and may conveniently be between 2 to 60% of the weight of a given unit dosage form. The amount of active compound in such therapeutically useful compositions is such that an effective dosage level will be obtained.

The tablets, troches, pills, and capsules, may also contain the following: binders such as gum tragacanth, acacia, corn starch or gelatin; excipients such as dicalcium phosphate; a disintegrating agent such as corn starch, potato starch, and alginic acid; a lubricant such as magnesium stearate; and a sweetening agent such as sucrose, fructose, lactose or aspartame or a flavoring agent such as peppermint, oil of wintergreen, or cherry flavoring may be added. When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac or sugar. A syrup or elixir may contain the active compound, sucrose or fructose as a sweetening agent, methyl and propylparabens as preservatives, a dye and flavoring such as cherry or orange flavor. Of course, any material used in preparing any unit dosage form should be pharmaceutically acceptable and substantially non-toxic in the amounts employed. In addition, the active compound may be incorporated into sustained-release preparations and devices.

The compounds or compositions can also be administered intravenously, or intraperitoneally by infusion or injection. Solutions of the active compound or its salts can be prepared in water, optionally mixed with a nontoxic surfactant. Dispersions can also be prepared in glycerol, liquid polyethylene glycols, triacetin, and mixtures thereof and in oils.

Useful dosages of the compounds of formula I can be determined by comparing their *in vitro* activity, and *in vivo* activity in animal models. Methods for the extrapolation of effective dosages in mice, and other animals, to humans are known to the art; for example, see U.S. Patent No. 4,938,949.

The compound can be administered in unit dosage form; for example, containing 0.05 mg to 500 mg, conveniently 0.1 mg to 250 mg, most conveniently, 1 mg to 150 mg of active ingredient per unit dosage form. The desired dose may be presented in a single dose or as divided doses administered at appropriate intervals.The compositions can be administered orally, sublingually, transdermally, or parenterally at dose levels of 0.01 to 150 mg/kg, preferably 0.1 to 50 mg/kg, and more preferably 0.1 to 30 mg/kg of mammal body weight.

### EXAMPLE: Effect of compounds on food intake in ob/ob mice

### Animals

Obese (ob/ob) mouse is selected as the primary animal model for screening as this mutant mouse consumes high amounts of food resulting in a high signal to noise ratio. To further substantiate and compare efficacy data, the effect of the compounds on food consumption is also studied in wild type (C57BL/6J) mice. The amount of food consumed during 15 hours of infusion of compounds is recorded.

Male mice (obese C57BL/6JBom-Lep^{ob} and lean wild-type C57B1/6JBom; Bomholtsgaard, Denmark) 8-9 weeks with an average body weight of 50 g (obese) and 25 g (lean) are used in all the studies. The animals are housed singly in cages at 23±1°C, 40-60 % humidity and have free access to water and standard laboratory chow. The 12/12-h light/dark cycle is set to lights off at 5 p.m. The animals are conditioned for at least one week before start of study.

### Compounds

The test compounds are dissolved in solvents suitable for each specific compound such as cyclodextrin, cyclodextrin/methane sulfonic acid, polyethylene glycol/methane sulfonic acid, or saline. Fresh solutions are made for each study. Doses of 30, 50 and 100 mg kg⁻¹day⁻¹ are used. The purity of the test compounds is of analytical grade.

### Minipump implantation

The animals are weighed at the start of the study and randomized based on body weight. Alzet osmotic minipumps (Model 2001D; infusion rate 8 µl/h) are used and loaded essentially as recommended by the Alzet technical information manual (Alza Scientific Products, 1997; Teeuwes and Yam, 1976). Continuous subcutaneous infusion with 24 hours duration is used. The minipumps are either filled with different concentrations of test compounds dissolved in vehicle or with only vehicle solution and maintained in vehicle pre-warmed to 37°C (approx. 1h). The minipumps are implanted subcutaneously in the neck/back region under short acting anesthesia (metofane/enflurane). This surgical procedure lasts approximately 5 min. It takes about 3 h to reach steady state delivery of the compound.

### Food intake measurements

The weights of the food pellets are measured at 5 p.m. and at 8 p.m. for two days before (baseline) and one day after the implantation of the osmotic minipumps. The weighing is performed with a computer assisted Mettler Toledo PR 5002 balance. Occasional spillage is corrected for. At the end of the study the animals are killed by neck dislocation and trunk blood sampled for later analysis of plasma drug concentrations.

### Determination of plasma concentration

The plasma sample proteins are precipitated with methanol, centrifuged and the supernatant is transferred to HPLC vials and injected into the liquid chromatography /mass spectrometric system. The mass spectrometer is set for electrospray positive ion mode and Multiple Reaction Monitoring (MRM with the transition m/z 316 ⇒ 221).

A linear regression analysis of the standards forced through the origin is used to calculate the concentrations of the unknown samples.

### Statistical evaluation

Food consumption for 15 hours is measured for the three consecutive days and the percentage of basal level values is derived for each animal from the day before and after treatment. The values are expressed as mean±SD and mean±SEM from eight animals per dose group. Statistical evaluation is performed by Kruskal-Wallis one-way ANOVA using the per cent basal values. If statistical significance is reached at the level of p<0.05, Mann-Whitney U-test for statistical comparison between control and treatment groups is performed.

### Results

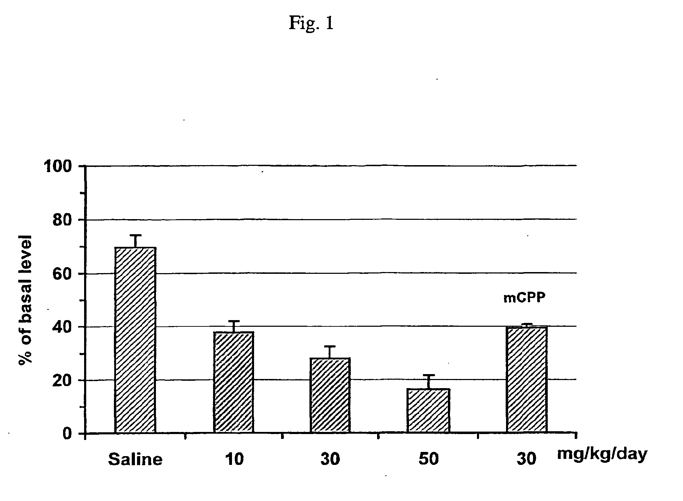

Fig. 1 shows the reduction of food intake, after subcutaneous continuos infusion of test compound SB-271046 (5-Chloro-3-methyl-benzo-[b]thiophene-2-sulphonic acid (4-methoxy-3-piperazin-yl-phenyl)-amide monohydrochloride) at the dose of 10, 30 and 50 mg/kg/day. The compound induced significant reduction of food intake of 45% (0.006*), 60 % (0.019*) and 77 % (0.034*) respectively compared to the basal level of food intake. *Free plasma concentration at the steady state giving the effect at the respective doses. m-Chloro-phenylpiperazine (mCPP) was used as a positive control.

## Claims

1. Use of a compound of formula (I) or (II) 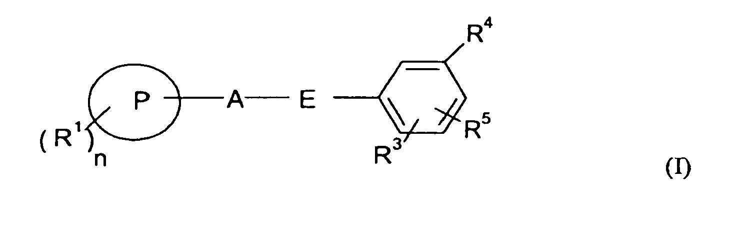 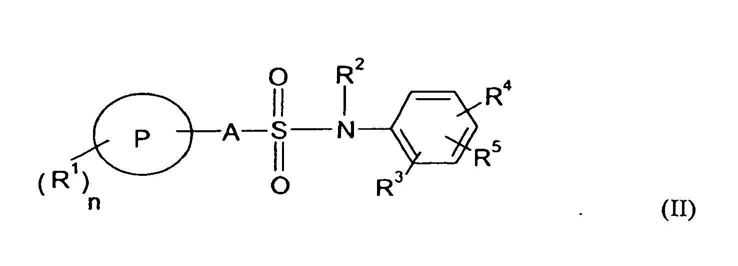 wherein
E is -SO₂NH- or -NHSO₂-;
R² is hydrogen, C₁₋₆ alkyl or arylC₁₋₆alkyl;
P is phenyl, naphthyl a bicyclic heterocyclic ring or is a 5- to 7-membered heterocyclic ring each containing 1 to 4 heteroatoms selected from oxygen, nitrogen or sulfur;
A is a single bond, a C₁₋₆ alkylene or a C₁₋₆ alkenylene group;
R¹ is halogen, C₁₋₆ alkyl, optionally substituted by one or more halogen atoms, C₃₋₆ cycloalkyl, COC₁₋₆ alkyl, C₁₋₆ alkoxy, OCF₃, hydroxy, hydroxy C₁₋₆alkyl, hydroxy C₁₋₆alkoxy, C₁₋₆ alkoxy C₁₋₆ alkoxy, C₁₋₆ alkanoyl, acyl, nitro, amino, alkylamino or dialkylamino, cyano or SR¹¹ where R¹¹ is hydrogen or C₁₋₆ alkyl or R¹ is phenyl, benzyl, naphthyl, a bicyclic heterocyclic ring, or is a 5 to 7-membered heterocyclic ring, each containing 1 to 4 heteroatoms selected from oxygen, nitrogen or sulfur,
n is 0, 1, 2, 3, 4, 5 or 6;
R³ is a group R⁵ or together with R⁵ forms a group (CH₂)₂O or (CH₂)₃O or R³ is linked to R² to form a group (CH₂)₂ or (CH₂)₃;
R⁴ is -X(CH₂)p-R⁶ where X is a single bond, CH₂, O, NH or N-C₁₋₆-alkyl and p is 0 to 6 and R⁶ is an optionally substituted 5- to 7-membered heterocyclic ring containing 1 to 3 heteroatoms selected from nitrogen, sulfur or oxygen, or R⁶ is NR⁷R⁸ where R⁷ and R⁸ are independently hydrogen, C₁₋₆ alkyl or aryl C₁₋₆ alkyl, or
R⁴ is selected from a group of formula (i), (ii) or (iii) 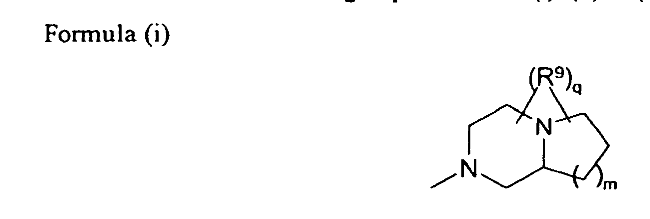
wherein R⁹ is C₁₋₆alkyl, or C₁₋₆alkyl substituted by one or more halogen atoms;
m is 0, 1 or 2;
q is 0, 1, 2, 3 or 4; or 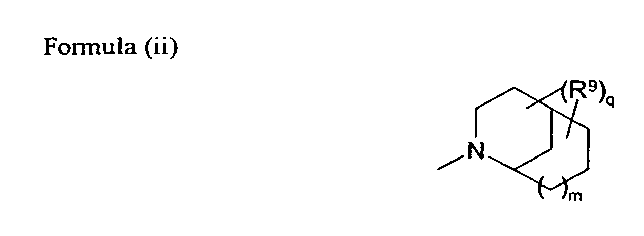
wherein R⁹, m and q are as defined in formula (i); or 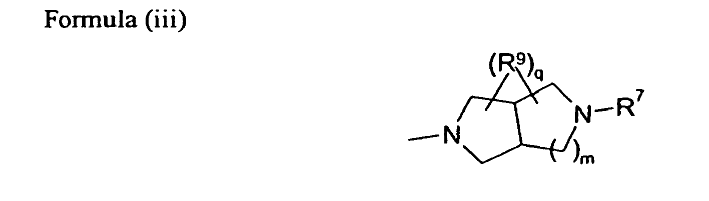 wherein R⁹, m and q are as defined in formula (i) and R⁷ is hydrogen or C₁₋₆alkyl;
R⁵ is hydrogen, halogen, C₁₋₆alkyl, C₁₋₆alkoxy, optionally substituted with one or more fluorine atoms, C₃₋₆ cycloalkyl, COC₁₋₆ alkyl, C₁₋₆ alkoxy, hydroxy, hydroxy C₁₋₆ alkyl, hydroxy C₁₋₆alkoxy, C₁₋₆ alkoxy -C₁₋₆ alkoxy, acyl, nitro, trifluoromethyl or together with R³ forms a group (CH₂)₂O or (CH₂)₃O, cyano or aryl;
or pharmaceutically acceptable salts thereof, in the manufacture of a medicament for the treatment of obesity.

2. The use according to claim 1 wherein
R¹ is halogen, C₁₋₆ alkyl, optionally substituted by one or more fluorine atoms, C₃₋₆ cycloalkyl, C₁₋₆ alkoxy, OCF₃, C₁₋₆ alkoxy C₁₋₆ alkoxy, C₁₋₆ alkanoyl, amino, alkylamino or dialkylamino, SR¹¹ where R¹¹ is hydrogen or C₁₋₆ alkyl or R¹ is phenyl, benzyl, naphthyl, a bicyclic heterocyclic ring, or is a 5 to 7-membered heterocyclic ring, each containing 1 to 4 heteroatoms selected from oxygen, nitrogen or sulfur;
n is 0, 1, 2, 3, 4 or 5;
R³ is a group R⁵ or together with R⁵ forms a group (CH₂)₂O or (CH₂)₃O;
R⁴ is selected from a group of formula (i), (ii) or (iii) as mentioned above;
R⁵ is hydrogen, halogen, C₁₋₆alkyl, C₁₋₆alkoxy, optionally substituted with one or more fluorine atoms, trifluoromethyl or together with R³ forms a group (CH₂)₂O or (CH₂)₃O in formula (I).

3. The use according to claim 1 wherein
R¹ is halogen, C₁₋₆alkyl optionally substituted by one or more halogen atoms, C₃₋₆ cycloalkyl, COC₁₋₆ alkyl, C₁₋₆alkoxy, OCF₃, hydroxy, hydroxy C₁₋₆alkyl, hydroxy C₁₋₆alkoxy, C₁₋₆alkoxyC₁₋₆alkoxy, acyl, nitro, amino, alkylamino or dialkylamino, cyano or R¹ is phenyl, naphthyl, a bicyclic heterocyclic ring or is a 5 to 7-membered heterocyclic ring, each containing 1 to 4 heteroatoms selected from oxygen, nitrogen or sulfur;
R⁴ is -X(CH₂)p-R⁶ where X is a single bond, CH₂, O, NH or N-C₁₋₆-alkyl and p is 0 to 6 and R⁶ is an optionally substituted 5- to 7-membered heterocyclic ring containing 1 to 3 heteroatoms selected from nitrogen, sulfur or oxygen, or R⁶ is NR⁷R⁸ where R⁷ and R⁸ are independently hydrogen, C₁₋₆ alkyl or aryl C₁₋₆ alkyl and
R⁵ is hydrogen, halogen, C₁₋₆ alkyl, C₃₋₆ cycloalkyl, COC₁₋₆ alkyl, C₁₋₆ alkoxy, hydroxy, hydroxy C₁₋₆alkyl, hydroxy C₁₋₆alkoxy, C₁₋₆alkoxyC₁₋₆alkoxy, acyl, nitro, trifluoromethyl, cyano or aryl in formula (II).

4. The use according to any one of claims 1 to 3 wherein P is benzothiophene.

5. The use according to any one of claims 1 to 4 wherein R¹ is halogen or C₁₋₆ alkyl optionally substituted with one or more halogen atoms.

6. The use according to any one of claims 1 to 5 wherein R² is hydrogen.

7. The use according to any one of claims 1 to 6 wherein R⁴ in formula (II) is an unsubstituted piperazine ring.

8. The use according to any one of claims 1 to 7 wherein R⁵ is C₁₋₆ alkoxy.

9. The use according to any one of claims 1 to 8 wherein P-A is 5-chloro-3-methylbenzo(2)thiophen-2-yl.

10. The use according to claim 1 wherein the compound is: 5-chloro-N-(4-methoxy-3-piperazin-1-yl-phenyl)-3-methyl-2-benzothiophenesulfonamide.

## Patentansprüche

1. Verwendung einer Verbindung der Formel (I) oder (II) 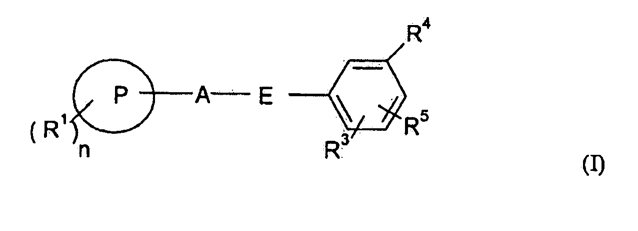 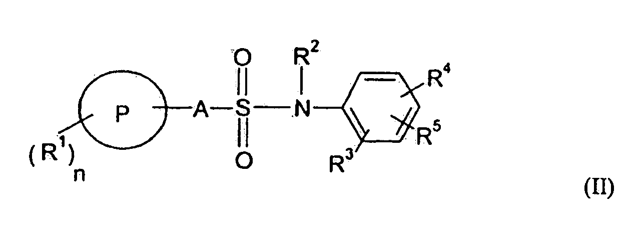 wobei
E -SO₂NH- oder -NHSO₂- ist;
R² Wasserstoff, C₁₋₆-Alkyl oder Aryl-C₁₋₆-Alkyl ist;
P Phenyl, Naphthyl, ein bicyclischer heterocyclischer Ring ist oder ein 5- bis 7-gliedriger heterocyclischer Ring ist, der jeweils 1 bis 4 Heteroatome enthält, die aus Sauerstoff, Stickstoff oder Schwefel ausgewählt sind;
A eine Einfachbindung, ein C₁₋₆-Alkylen oder eine C₁₋₆-Alkenylengruppe ist;
R¹ Halogen, C₁₋₆-Alkyl, wahlweise durch ein oder mehr Halogenatome substituiert, C₃₋₆-Cycloalkyl, COC₁₋₆-Alkyl, C₁₋₆-Alkoxy, OCF₃, Hydroxy, Hydroxy-C₁₋₆-Alkyl, Hydroxy-C₁₋₆-Alkoxy, C₁₋₆-Alkoxy-C₁₋₆-Alkoxy, C₁₋₆Alkanoyl, Acyl, Nitro, Amino, Alkylamino oder Dialkylamino, Cyano oder SR¹¹ ist, worin R¹¹ Wasserstoff oder C₁₋₆₋Alkyl ist, oder R¹ Phenyl, Benzyl, Naphthyl, ein bicyclischer heterocyclischer Ring ist oder ein 5- bis 7-gliedriger heterocyclischer Ring ist, der jeweils 1 bis 4 Heteroatome enthält, die aus Sauerstoff, Stickstoff oder Schwefel ausgewählt sind;
n 0, 1, 2, 3, 4, 5 oder 6 ist;
R³ eine Gruppe R⁵ ist oder zusammen mit R⁵ eine Gruppe (CH₂)₂O oder (CH₂)₃O bildet oder R³ mit R² zu einer Gruppe (CH₂)₂ oder (CH₂)₃ verbunden ist;
R⁴ 4-X(CH₂)p-R⁶ ist, worin X eine Einfachbindung, CH₂, O, NH oder N-C₁₋₆-Alkyl ist und p 0 bis 6 ist und R⁶ ein wahlweise substituierter, 5- bis 7-gliedriger heterocyclischer Ring ist, der 1 bis 3 Heteroatome enthält, die aus Stickstoff, Schwefel oder Sauerstoff ausgewählt sind, oder R⁶ NR⁷R⁸ ist, worin R⁷ und R⁸ unabhängig voneinander Wasserstoff, C₁₋₆-Alkyl oder Aryl-C₁₋₆-Alkyl sind, oder R⁴ aus einer Gruppe der Formel (i), (ii) oder (iii) ausgewählt ist 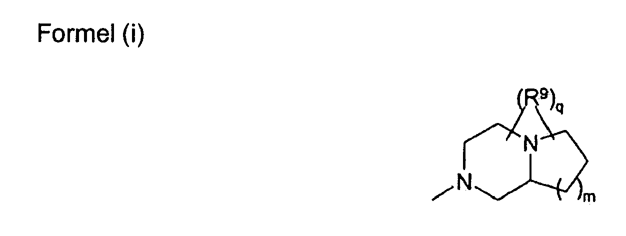
worin R⁹ C₁₋₆-Alkyl oder C₁₋₆-Alkyl, das durch ein oder mehr Halogenatome substituiert ist, ist;
m 0, 1 oder 2 ist;
q 0, 1, 2, 3 oder 4 ist; oder 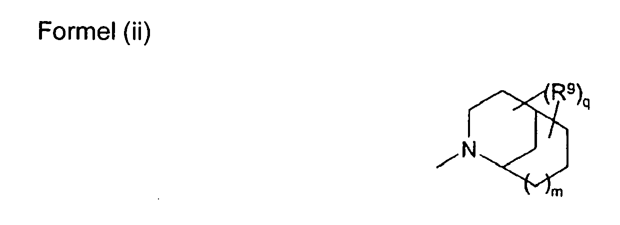
worin R⁹, m und q wie in der Formel (i) definiert sind; oder 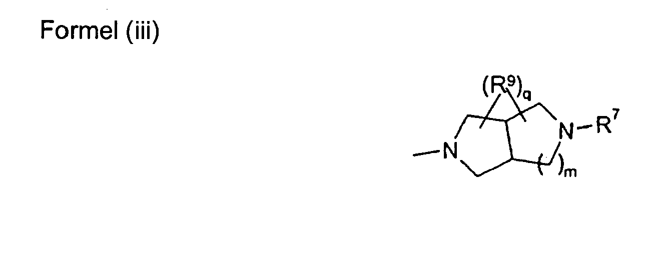 worin R⁹, m und q wie in der Formel (i) definiert sind und R⁷ Wasserstoff oder C₁₋₆₋Alkyl ist;
R⁵ Wasserstoff, Halogen, C₁₋₆-Alkyl, C₁₋₆-Alkoxy, wahlweise mit einem oder mehr Fluoratomen substituiert, C₃₋₆-Cycloalkyl, COC₁₋₆-Alkyl, C₁₋₆-Alkoxy, Hydroxy, Hydroxy-C₁₋₆-Alkyl, Hydroxy-C₁₋₆-Alkoxy, C₁₋₆-Alkoxy-C₁₋₆-Alkoxy, Acyl, Nitro, Trifluormethyl ist oder zusammen mit R³ eine Gruppe (CH₂)₂O oder (CH₂)₃O, Cyano oder Aryl bildet;
oder von pharmazeutisch annehmbaren Salzen davon zur Herstellung eines Medikaments zur Behandlung von Fettsucht.

2. Verwendung nach Anspruch 1, wobei
R¹ Halogen, C₁₋₆-Alkyl, wahlweise durch ein oder mehr Fluoratome substituiert, C₃₋₆₋Cycloalkyl, C₁₋₆-Alkoxy, OCF₃, C₁₋₆-Alkoxy-C₁₋₆-Alkoxy, C₁₋₆-Alkanoyl, Amino, Alkylamino oder Dialkylamino, SR¹¹ ist, worin R¹¹ Wasserstoff oder C₁₋₆-Alkyl ist, oder R¹ Phenyl, Benzyl, Naphthyl, ein bicyclischer heterocyclischer Ring ist oder ein 5- bis 7-gliedriger heterocyclischer Ring ist, der jeweils 1 bis 4 Heteroatome enthält, die aus Sauerstoff, Stickstoff oder Schwefel ausgewählt sind;
n 0, 1, 2, 3, 4 oder 5 ist;
R³ eine Gruppe R⁵ ist oder zusammen mit R⁵ eine Gruppe (CH₂)₂O oder (CH₂)₃O bildet;
R⁴ aus der Gruppe der Formel (i), (ii) oder (iii), wie oben angegeben, ausgewählt ist;
R⁵ Wasserstoff, Halogen, C₁₋₆-Alkyl, C₁₋₆-Alkoxy, wahlweise mit einem oder mehr Fluoratomen substituiert, Trifluormethyl ist oder zusammen mit R³ eine Gruppe (CH₂)₂O oder (CH₂)₃O in der Formel (I) bildet.

3. Verwendung nach Anspruch 1, wobei
R¹ Halogen, C₁₋₆-Alkyl, wahlweise mit einem oder mehr Halogenatomen substituiert, C₃₋₆-Cycloalkyl, COC₁₋₆-Alkyl, C₁₋₆-Alkoxy, OCF₃, Hydroxy, Hydroxy-C₁₋₆-Alkyl, Hydroxy-C₁₋₆-Alkoxy, C₁₋₆-Alkoxy-C₁₋₆-Alkoxy, Acyl, Nitro, Amino, Alkylamino oder Dialkylamino, Cyano ist oder R¹ Phenyl, Naphthyl, ein bicyclischer heterocyclischer Ring ist oder ein 5- bis 7-gliedriger heterocyclischer Ring ist, der jeweils 1 bis 4 Heteroatome enthält, die aus Sauerstoff, Stickstoff oder Schwefel ausgewählt sind;
R⁴ -X(CH₂)p-R⁶ ist, worin X eine Einfachbindung, CH₂, O, NH oder N-C₁₋₆-Alkyl ist und p 0 bis 6 ist und R⁶ ein wahlweise substituierter, 5- bis 7-gliedriger heterocyclischer Ring ist, der 1 bis 3 Heteroatome enthält, die aus Stickstoff, Schwefel oder Sauerstoff ausgewählt sind, oder R⁶ NR⁷R⁸ ist, worin R⁷ und R⁸ unabhängig voneinander Wasserstoff, C₁₋₆-Alkyl oder Aryl-C₁₋₆-Alkyl sind, und
R⁵ Wasserstoff, Halogen, C₁₋₆-Alkyl, C₃₋₆-Cycloalkyl, COC₁₋₆-Alkyl, C₁₋₆-Alkoxy, Hydroxy, Hydroxy-C₁₋₆-Alkyl, Hydroxy-C₁₋₆-Alkoxy, C₁₋₆-Alkoxy-C₁₋₆-Alkoxy, Acyl, Nitro, Trifluormethyl, Cyano oder Aryl in der Formel (II) bildet.

4. Verwendung nach einem der Ansprüche 1 bis 3, wobei P Benzothiophen ist.

5. Verwendung nach einem der Ansprüche 1 bis 4, wobei R¹ Halogen oder C₁₋₆₋Alkyl, wahlweise mit einem oder mehr Halogenatomen substituiert, ist.

6. Verwendung nach einem der Ansprüche 1 bis 5, wobei R² Wasserstoff ist.

7. Verwendung nach einem der Ansprüche 1 bis 6, wobei R⁴ in der Formel (II) ein unsubstituierter Piperazinring ist.

8. Verwendung nach einem der Ansprüche 1 bis 7, wobei R⁵ C₁₋₆-Alkoxy ist.

9. Verwendung nach einem der Ansprüche 1 bis 8, wobei P-A 5-Chlor-3-methylbenzo[2]thiophen-2-yl ist.

10. Verwendung nach Anspruch 1, wobei die Verbindung 5-Chlor-N-(4-methoxy-3-piperazin-1-yl-phenyl)-3-methyl-2-benzothiophensulfonamid ist.

## Revendications

1. Utilisation d'un composé de formule (I) ou (II) 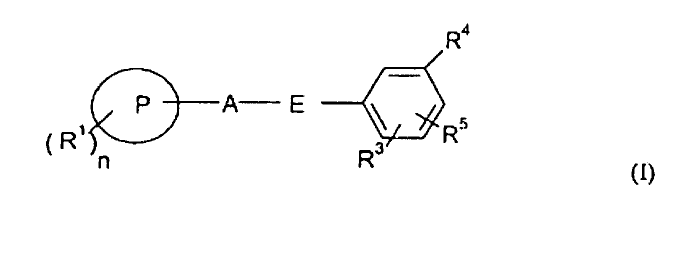 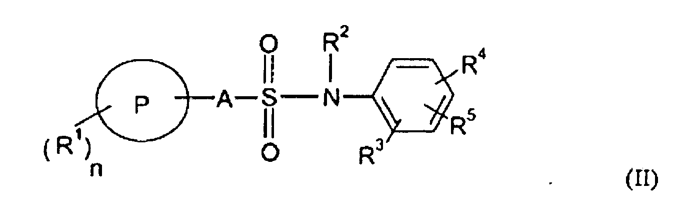 où
E est -SO₂NH- ou -NHSO₂- ;
R² est un hydrogène, un alkyle en C₁-C₆ ou un aryl-(alkyle en C₁-C₆) ;
P est un phényle, un naphtyle, un cycle hétérocyclique bicyclique ou est un cycle hétérocyclique à 5 à 7 éléments contenant chacun 1 à 4 hétéroatomes choisis parmi l'oxygène, l'azote ou le soufre ;
A est une liaison simple, un alkylène en C₁-C₆ ou un groupement alkénylène en C₁-C₆;
R¹ est un halogène, un alkyle en C₁-C₆, optionnellement substitué par un ou plusieurs atomes d'halogène, un cycloalkyle en C₃-C₆, un CO-(alkyle en C₁-C₆), un alkoxy en C₁-C₆, OCF₃, un hydroxy, un hydroxy-(alkyle en C₁-C₆), un hydroxy-(alkoxy en C₁-C₆), un (alkoxy en C₁-C₆)-(alkoxy en C₁-C₆), un alkanoyle en C₁-C₆, un acyle, un nitro, un amino, un alkylamino ou un dialkylamino, un cyano ou SR¹¹ où R¹¹ est un hydrogène ou un alkyle en C₁-C₆ ou R¹ est un phényle, un benzyle, un naphtyle, un cycle hétérocyclique bicyclique ou est un cycle hétérocyclique à 5 à 7 éléments, contenant chacun 1 à 4 hétéroatomes choisis parmi l'oxygène, l'azote ou le soufre ;
n est 0, 1, 2, 3, 4, 5 ou 6 ;
R³ est un groupement R⁵ ou ensemble avec R⁵ forme un groupement (CH₂)₂O ou (CH₂)₃O ou R³ est lié à R² pour former un groupement (CH₂)₂ ou (CH₂)₃ ;
R⁴ est -X(CH₂)p-R⁶ où X est une liaison simple, CH₂, O, NH ou N-(alkyle en C₁-C₆) et p est 0 à 6 et R⁶ est un cycle hétérocyclique à 5 à 7 éléments optionnellement substitué, contenant 1 à 3 hétéroatomes choisis parmi l'azote, le soufre ou l'oxygène, ou R⁶ est NR⁷R⁸ où R⁷ et R⁸ sont indépendamment un hydrogène, un alkyle en C₁-C₆ ou un aryl-(alkyle en C₁-C₆), ou
R⁴ est choisi parmi un groupe de formule (i), (ii) ou (iii) 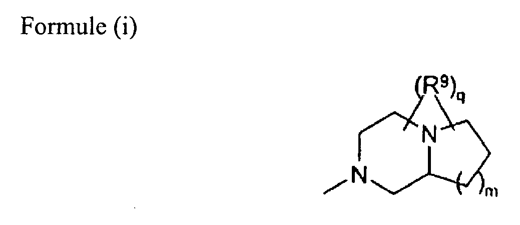
où R⁹ est un alkyle en C₁-C₆ ou un alkyle en C₁-C₆ substitué par un ou plusieurs atomes d'halogène ;
m est 0, 1 ou 2 ;
q est 0, 1, 2, 3 ou 4 ; ou 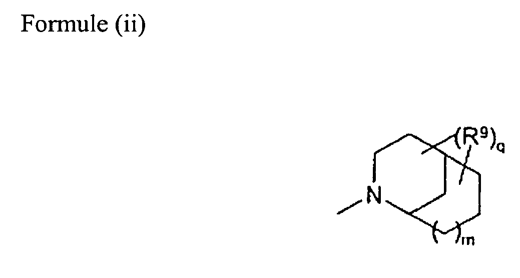
où R⁹, m et q sont tels que définis dans la formule (i) ; ou 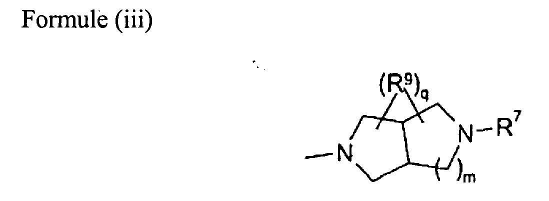 où R⁹, m et q sont tels que définis dans la formule (i) et R⁷ est un hydrogène ou un alkyle en C₁-C₆ ;
R⁵ est un hydrogène, un halogène, un alkyle en C₁-C₆, un alkoxy en C₁-C₆, optionnellement substitué par un ou plusieurs atomes de fluor, un cycloalkyle en C₃-C₆, un CO-(alkyle en C₁-C₆), un alkoxy en C₁-C₆, un hydroxy, un hydroxy-(alkyle en C₁-C₆), un hydroxy-(alkoxy en C₁-C₆), un (alkoxy en C₁-C₆)-(alkoxy en C₁-C₆), un acyle, un nitro, un trifluorométhyle ou ensemble avec R³ forme un groupement (CH₂)₂O ou (CH₂)₃O, un cyano ou un aryle ;
ou de sels pharmaceutiquement acceptables de ceux-ci, pour la fabrication d'un médicament destiné au traitement de l'obésité.

2. Utilisation selon la revendication 1, dans laquelle
R¹ est un halogène, un alkyle en C₁-C₆, optionnellement substitué par un ou plusieurs atomes de fluor, un cycloalkyle en C₃-C₆, un alkoxy en C₁-C₆, OCF₃, un (alkoxy en C₁-C₆)-(alkoxy en C₁-C₆), un alkanoyle en C₁-C₆, un amino, un alkylamino ou un dialkylamino, SR¹¹ où R¹¹ est un hydrogène ou un alkyle en C₁-C₆ ou R¹ est un phényle, un benzyle, un naphtyle, un cycle hétérocyclique bicyclique ou est un cycle hétérocyclique à 5 à 7 éléments, contenant chacun 1 à 4 hétéroatomes choisis parmi l'oxygène, l'azote ou le soufre ;
n est 0, 1, 2, 3, 4 ou 5;
R³ est un groupement R⁵ ou ensemble avec R⁵ forme un groupement (CH₂)₂O ou (CH₂)₃O,
R⁴ est choisi parmi un groupe de formule (i), (ii) ou (iii) telle que mentionnée plus haut ;
R⁵ est un hydrogène, un halogène, un alkyle en C₁-C₆, un alkoxy en C₁-C₆, optionnellement substitué par un ou plusieurs atomes de fluor, un trifluorométhyle ou ensemble avec R³ forme un groupement (CH₂)₂O ou (CH₂)₃O dans la Formule (I).

3. Utilisation selon la revendication 1, dans laquelle
R¹ est un halogène, un alkyle en C₁-C₆, optionnellement substitué par un ou plusieurs atomes d'halogène, un cycloalkyle en C₃-C₆, un CO-(alkyle en C₁-C₆), un alkoxy en C₁-C₆, OCF₃, un hydroxy, un hydroxy-(alkyle en C₁-C₆), un hydroxy-(alkoxy en C₁-C₆), un (alkoxy en C₁-C₆)-(alkoxy en C₁-C₆), un acyle, un nitro, un amino, un alkylamino ou un dialkylamino, un cyano ou R¹ est un phényle, un naphtyle, un cycle hétérocyclique bicyclique ou est un cycle hétérocyclique à 5 à 7 éléments, contenant chacun 1 à 4 hétéroatomes choisis parmi l'oxygène, l'azote ou le soufre ;
R⁴ est -X(CH₂)p-R⁶ où X est une liaison simple, CH₂, O, NH ou N-(alkyle en C₁-C₆) et p est 0 à 6 et R⁶ est un cycle hétérocyclique à 5 à 7 éléments optionnellement substitué, contenant 1 à 3 hétéroatomes choisis parmi l'azote, le soufre ou l'oxygène, ou R⁶ est NR⁷R⁸ où R⁷ et R⁸ sont indépendamment un hydrogène, un alkyle en C₁-C₆ ou un aryl-(alkyle en C₁-C₆), et
R⁵ est un hydrogène, un halogène, un alkyle en C₁-C₆, un cycloalkyle en C₃-C₆, un CO-(alkyle en C₁-C₆), un alkoxy en C₁-C₆, un hydroxy, un hydroxy-(alkyle en C₁-C₆), un hydroxy-(alkoxy en C₁-C₆), un (alkoxy en C₁-C₆)-(alkoxy en C₁₋C₆), un acyle, un nitro, un trifluorométhyle, un cyano ou un aryle dans la formule (II).

4. Utilisation selon l'une quelconque des revendications 1 à 3 dans laquelle P est un benzothiophène.

5. Utilisation selon l'une quelconque des revendications 1 à 4 dans laquelle R¹ est un halogène ou un alkyle en C₁-C₆ optionnellement substitué par un ou plusieurs atomes d'halogène.

6. Utilisation selon l'une quelconque des revendications 1 à 5 dans laquelle R² est un hydrogène.

7. Utilisation selon l'une quelconque des revendications 1 à 6 dans laquelle R⁴ dans la Formule (II) est un cycle pipérazine non substitué.

8. Utilisation selon l'une quelconque des revendications 1 à 7 dans laquelle R⁵ est un alkoxy en C₁-C₆.

9. Utilisation selon l'une quelconque des revendications 1 à 8 dans laquelle P-A est 5-chloro-3-méthyl-benzo[2]thiophen-2-yle.

10. Utilisation selon la revendication 1 dans laquelle le composé est : 5-chloro-N-(4-méthoxy-3-pipérazin-1-yl-phényl)-3-méthyl-2-benzothiophènesulfonamide.
